# EUROPÄISCHE PATENTSCHRIFT

(11) **EP 3 927 264 B1**
(45) Veröffentlichungstag und Bekanntmachung des Hinweises auf die Patenterteilung: **22.03.2023**
(21) Anmeldenummer: 20706697.8
(22) Anmeldetag: 20.02.2020
(51) Int. Cl.: A61B 17/86, A61B 17/04, A61B 17/88, A61F 2/08

(54) **KORTIKALISSCHRAUBE AUS BIORESORBIERBAREM MATERIAL**
CORTICALIS SCREW CONSISTING OF BIORESORBABLE MATERIAL
VIS CORTICALE EN MATÉRIAU BIORÉSORBABLE

(30) Priorität: 22.02.2019 DE 102019104545
(43) Veröffentlichungstag der Anmeldung: 29.12.2021
(73) Patentinhaber: Botiss Biomaterials GmbH, 15806 Zossen (DE)
(72) Erfinder: BIELENSTEIN, Oliver, 10629 Berlin (DE); WITTE, Frank, 10589 Berlin (DE); MATTHYS, Romano, 8810 Horgen (CH); DR. TADIC, Drazen, 14129 Berlin (DE)
(74) Vertreter: Friderichs, Gunther
(86) Internationale Anmeldenummer: PCT/EP2020/054484
(87) Internationale Veröffentlichungsnummer: WO 2020/169735

(56) Entgegenhaltungen:
- EP-A1- 3 072 540
- EP-A1- 3 315 085
- EP-A2- 1 234 637
- FR-A1- 2 803 739
- FR-A1- 2 879 915
- FR-B1- 2 803 739
- FR-B1- 2 879 915
- US-A- 5 695 497
- US-A1- 2001 041 937
- US-A1- 2005 177 243
- US-A1- 2012 179 163
- US-B1- 6 283 973

## Beschreibung

### Gebiet der Erfindung

Die Erfindung betrifft eine Kortikalisschraube aus einem bioresorbieren Material sowie ein Set mit einer Kortikalisschraube.

### Hintergrund der Erfindung

Kortikalisschrauben sind aus der Praxis in verschiedensten Ausführungsbeispielen bekannt.

Diese sind insbesondere zur Fixierung von Ligamenten, Knochenfragment sowie zur Befestigung von Implantaten, insbesondere von Knochenplatten, ausgebildet.

In der Regel umfasst eine Kortikalisschraube ein Schraubengewinde zum Eindrehen in die Kortikalis sowie einen Kopf mit einem Antrieb.

Aus der Praxis bekannt sind auch bioresorbierbare Schrauben.

Diese können insbesondere aus einem bioresorbierbaren Metall bestehen. Insbesondere die Verwendung von Magnesium als bioresorbierbares Material ist seit langem bekannt.

Da reines Magnesium keine sehr hohe Festigkeit aufweist und in der Regel sehr schnell korrodiert, werden in der Praxis zumeist Magnesiumlegierungen verwendet. So sind insbesondere yttriumhaltige Magnesiumlegierungen bekannt, welche eine höhere Festigkeit als reines Magnesium aufweisen und wobei vor allem aufgrund des Yttriums die Korrosion des Implantats herabgesetzt ist.

Aus der Praxis bekannte Knochenschrauben aus einer Magnesiumlegierung umfassen einen Kopf mit einem Antrieb, welcher beispielsweise als Innensechsrund ausgebildet ist.

Problematisch ist, dass die verwendeten Magnesiumlegierungen immer noch eine deutlich geringere Festigkeit als herkömmlich verwendete Titan- oder Edelstahllegierungen aufweisen. Insbesondere bei kleineren Implantaten mit einem Durchmesser von weniger als 5 mm besteht daher die Gefahr, dass der Antrieb beim Hereindrehen des Implantats beschädigt wird.

Ein weiteres, aus der Praxis hinlänglich bekanntes Problem ist die Wasserstoffbildung beim Abbau. Gasblasenbildungen können den Heilungsprozess beeinträchtigen.

Es ist daher angestrebt, derartige Implantate aus möglichst wenig Material auszubilden. Hohlstrukturen bei einer Schraube, insbesondere eine poröse Ausgestaltung des Materials, gehen jedoch wiederum mit einer derart verringerten Festigkeit einher, dass die Schraube unter Umständen beim Eindrehen innerhalb des Gewindebereichs zerbricht.

Medizinische Schrauben sind aus folgenden Dokumenten bekannt:
- EP 1 234 637 A2
- US 5 659 497 A
- FR 2 803 739 A1
- US 6 283 973 B1
- FR 2 879 915 A1
- US 2001/041937 A1
- US 2012/179163 A1
- EP 3 315 085 A1
- US 2005/177243 A1
- EP 3 072 540 A1

### Aufgabe der Erfindung

Der Erfindung liegt demgegenüber die Aufgabe zugrunde, eine Kortikalisschraube bereitzustellen, bei welcher die genannten Nachteile zumindest verringert sind.

Es ist insbesondere eine Aufgabe der Erfindung, eine Kortikalisschraube bereitzustellen, welche bei geringem Gewicht eine geringe Bruchgefahr während des Eindrehens hat.

### Zusammenfassung der Erfindung

Die Aufgabe der Erfindung wird bereits durch eine Kortikalisschraube nach Anspruch 1 gelöst.

Bevorzugte Ausführungsformen und Weiterbildungen der Erfindung sind dem Gegenstand der abhängigen Ansprüche, der Beschreibung sowie den Zeichnungen zu entnehmen.

Die Erfindung betrifft eine Kortikalisschraube aus einem bioresorbierbaren Material. Erfindungsgemäß besteht die Kortikalisschraube aus Magnesium oder einer Magnesiumlegierung.

Die Kortikalisschraube umfasst ein Schraubengewinde zum Eindrehen in die Kortikalis sowie einen Kopf mit einem Antrieb.

Der Antrieb ist also über den Kopf der Kortikalisschraube durch ein Handhabungswerkzeug zugänglich. Insbesondere kann der Antrieb ein Formschlusselement umfassen, welches sich axial durch den Kopf erstreckt.

Gemäß der Erfindung erstreckt sich der Antrieb zumindest abschnittsweise durch das Schraubengewinde.

Vorzugsweise erstreckt sich der Antrieb über zumindest die Hälfte des Schraubengewindes, ganz besonders bevorzugt durch das gesamte Schraubengewinde.

Der Erfindung liegt der Effekt zugrunde, dass durch die Bereitstellung eines durchgehenden Kanals vorzugsweise die gesamte Länge des Implantats zur Drehmomentübertragung genutzt werden kann. Gleichzeitig wird durch den Kanal das Gewicht der Kortikalisschraube wiederum reduziert, was bei Ausgestaltung der Kortikalisschraube aus Magnesium oder einer Magnesiumlegierung die Gasbildung beim Abbau verringert.

Das Handhabungswerkzeug, dessen Querschnitt vorzugsweise im Wesentlichen dem Querschnitt des Kanals entspricht, stabilisiert die Kortikalisschraube beim Eindrehen, so dass trotz eines, gegenüber einer Schraube ohne Kanal, leichteren Implantats das Risiko eines Brechens während des Eindrehens herabgesetzt ist.

Vorzugsweise ist der Antrieb als ein von einem hinteren Ende bis zu einem vorderen Ende durchgehenden Kanal ausgebildet ist, welcher ein Formschlusselement für ein Handhabungswerkzeug ausbildet.

Gemäß einer Ausführungsform weist das Schraubengewinde der Kortikalisschraube Durchgänge zum Kanal aufweist.

Der Kanal ist also seitlich an zumindest einer, vorzugsweise an mehreren Stellen geöffnet. Die Durchgänge dienen dem Druckausgleich. So kann zum einen ein Gasaustausch stattfinden, insbesondere da sich möglicherweise im Inneren des Kanals bildende Gase leicht entweichen.

Weiter können auch osmotische Drücke aufgrund der Bildung von Metallsalzen beim Abbau durch die Durchgänge in verbesserter Weise ausgeglichen werden.

Gemäß einer bevorzugten Ausführungsform der Erfindung sind die Durchgänge als Überschneidungen des Kanals mit dem Zahngrund eines Gewindes ausgebildet.

So lassen sich die Durchgänge beispielsweise auf sehr einfache Weise bereitstellen, indem ein Halbzeug mit dem Kanal mit dem Gewinde versehen wird. Der Zahngrund des Gewindes überschneidet sich dabei mit dem Kanal, so dass im Zahngrund Durchgänge entstehen.

Insbesondere bei Vorhandensein eines zentrischen kreuzförmigen Kanals bilden sich in jedem Gewindegang vier um jeweils 90° verteilte Durchgänge.

Vorzugsweise ist, insbesondere wie vorstehend beschrieben, der Kanal gleichzeitig als Antrieb für die Kortikalisschraube ausgebildet.

Gemäß der Erfindung geht das Schraubengewinde übergangslos in einen konischen Kopf mit einem Kopfgewinde übergeht. Unter einem übergangslosen Übergang wird verstanden, dass der letzte Gewindezug des Schraubengewindes in einen Gewindezug des Kopfgewindes übergeht.

Diese Ausgestaltung ermöglicht ein Verklemmen des Kopfes. Die Kortikalisschraube ist daher insbesondere zum Befestigen von Ligamenten oder Knochenfragmenten geeignet.

Gemäß der Erfindung hat das Schraubengewinde der Kortikalisschraube eine größere Steigung als das Kopfgewinde. Am Ende des Eindrehvorgangs verklemmt sich so die Kortikalisschraube nicht nur aufgrund des konischen Kopfes, sondern auch aufgrund der unterschiedlichen Gewindesteigungen.

Die Kortikalisschraube kann aus einer Magnesiumlegierung bestehen, welche 0,5 bis 10 Gewichts% Yttrium umfasst.

Die Kortikalisschraube umfasst vorzugsweise einen Kanal mit einem nicht kreisförmigen Querschnitt, so dass über ein Handhabungswerkzeug ein Drehmoment auf die Schraube ausgeübt werden kann.

Der Kanal hat vorzugsweise zumindest im Bereich eines Gewindes, besonders bevorzugt über seine gesamte Länge, einen uniformen Querschnitt.

Vorzugsweise verläuft der Kanal konzentrisch zur Mittelachse der Kortikalisschraube.

Der Kanal ist vorzugsweise vorn, also an der Spitze der Kortikalisschraube, offen.

Denkbar ist aber gemäß einer anderen Ausführungsform der Erfindung auch, einen durchgehenden Kanal bereitzustellen, welcher an einer Spitze, die beispielsweise als Bohrer mit Schneiden ausgebildet ist, endet. Der durchgehende Kanal reicht aber vorzugsweise zumindest als Sackloch bis zur Spitze hin und erstreckt sich insbesondere entlang eines Gewindes des Implantats.

Ein vorn offener Kanal hat insbesondere aber den Vorteil, dass die Kortikalisschraube auf effiziente Weise aus einer extrudierten Magnesiumlegierung hergestellt werden kann, bei welcher der als Formschlusselement dienende Kanal während des Extrusionsprozesses eingebracht wurde.

Gemäß einer bevorzugten Ausführungsform der Erfindung ist der Kanal kreuzförmig ausgebildet. Insbesondere besteht der Kanal, im Querschnitt gesehen, aus zwei sich überschneidenden Rechtecken, welche vorzugsweise identisch zueinander ausgebildet sind und wobei vorzugsweise die Mittelpunkte der Rechtecke aufeinander liegen.

Ein derartiger kreuzförmiger Querschnitt kann auf einfache Weise auch bei kleineren Durchmessern durch ein Extrusionsverfahren hergestellt werden und stellt vorzugsweise gleichzeitig lediglich zur Mittelachse versetzte radial verlaufende Seitenflächen bereit, welche als radial ausgerichtete Angriffsflächen für das Handhabungswerkzeug dienen.

Bei einer Weiterbildung der Erfindung umfasst die Kortikalisschraube eine Beschichtung.

Insbesondere umfasst die Kortikalisschraube eine Magnesiumfluorid-Beschichtung. Eine derartige Schicht kann durch Eintauchen der Kortikalisschraube in Flusssäure auf einfache Weise bereitgestellt werden.

Weiter kann die Kortikalisschraube, insbesondere um eine Korrosion unmittelbar nach dem Einsetzen zu verlangsamen, eine Polymerbeschichtung umfassen.

Es ist insbesondere vorgesehen, die Kortikalisschraube mit einer Kombination aus Magnesiumfluorid-Beschichtung und Polymerbeschichtung zu versehen. Dabei dient die Magnesiumfluorid-Beschichtung unter anderem als Haftvermittlerschicht.

Die Beschichtung, insbesondere die Polymerbeschichtung, hat vorzugsweise eine Dicke von weniger als 5 µm, vorzugsweise weniger als 1 µm, insbesondere eine Dicke von 0,2 bis 0,8 µm.

Durch eine derartige dünne Beschichtung wird die Gefahr von Rissbildung der Schicht beim Einsetzen minimiert.

Vorzugsweise hat die Kortikalisschraube eine Länge von 6 bis 20 mm und/oder einen Durchmesser von 2 bis 5 mm.

Gemäß einer weiteren Ausführungsform hat die Kortikalisschraube eine selbstschneidende Spitze. Eine derartige selbstschneidende Spitze kann auch bei einer Kortikalisschraube mit einem vorne offenen Kanal durch zumindest einen Anschliff zumindest des ersten vorderen Gewindegangs bereitgestellt werden.

Die selbstschneidende Spitze kann insbesondere durch eine Ausnehmung gebildet sein, die in der Draufsicht auf die Spitze der Kortikalisschraube im Wesentlichen die Form eines Kreissegments hat.

Die Ausnehmung läuft vorzugsweise im ersten Gewindegang oder nach dem ersten Gewindegang nach außen aus.

Die Kortikalisschraube kann aus einer Magnesiumlegierung mit einer Zugfestigkeit Rm (nach DIN EN ISO 6892-1, Version 2017-02) von über 250 MPa, insbesondere über 350 MPa, und/oder unter 500 MPa, insbesondere unter 450 MPa, bestehen.

Der Kanal der Kortikalisschraube ist insbesondere als Kreuz ausgebildet, welches im Querschnitt aus zwei sich senkrecht überlappenden, identischen Rechtecken gebildet wird.

Vorzugsweise sind die inneren und/oder äußeren Kanten des Kreuzes abgerundet ausgebildet. So kann die Rissgefahr an Kanten reduziert werden. Gleichzeitig wird durch die Abrundungen das Einbringen des Handhabungswerkzeugs erleichtert.

Vorzugsweise weist das Kreuz im Querschnitt eine Höhe und/oder eine Breite von 20 - 60 %, vorzugsweise 30 - 45 % und/oder abgerundete Kanten mit einem Kantenradius von 5 - 40 %, vorzugsweise 10 - 30 % des Außendurchmessers des Gewindes des Implantats auf.

Durch diese Dimensionierung konnte eine optimale Stabilisierung durch den Antrieb bei gleichzeitig wenig Materialvolumen erreicht werden.

Das Kreuz hat vorzugsweise im Querschnitt eine Höhe und/oder Breite von 1,3 bis 2,5 mm, besonders bevorzugt von 1,5 bis 2,1 mm.

Die abgerundeten Kanten haben vorzugsweise einen Kantenradius von 0,1 bis 0,8 mm, besonders bevorzugt von 0,2 bis 0,6 mm.

Die Erfindung betrifft des Weiteren ein Set mit der vorstehend beschriebenen Kortikalisschraube und einem Handhabungswerkzeug.

Das Handhabungswerkzeug ist insbesondere als Schraubendreher mit einem Griff oder als motorisch angetriebenes medizinisches Instrument ausgebildet.

Das Handhabungswerkzeug umfasst eine Welle, welche an einen als Formschlusselement ausgebildeten Kanal der Kortikalisschraube angepasst ist.

Die Welle hat insbesondere die gleiche Form wie der Kanal. Von den Toleranzen her sind Welle und Kanal vorzugsweise derart aufeinander abgestimmt, dass die Kortikalisschraube derart klemmend auf das Handhabungswerkzeug aufgeschoben werden kann, so dass diese beim Einsetzen gegen versehentliches Herabfallen gesichert ist.

Ein derartiges Klemmen kann insbesondere auf einfache Weise realisiert werden, da über den durchgehenden Kanal im Gegensatz zu einer herkömmlichen Schraube große Flächen bereitgestellt werden, über die eine reibschlüssige Verbindung hergestellt werden kann.

Die Welle des Handhabungswerkzeugs erstreckt sich vorzugsweise bis zum vorderen Ende der Kortikalisschraube.

Gemäß einer Weiterbildung der Erfindung ist die Spitze der Welle als Bohrer ausgebildet.

Diese Ausführungsform betrifft also Schrauben mit vorne offenem Kanal, wobei die Welle mit einem beispielsweise zum Bohren angespitzten Abschnitt vorne aus der Kortikalisschraube herausragt.

Das Handhabungswerkzeug kann so gleichzeitig zum Vorbohren oder Erweitern einer bereits gesetzten Vorbohrung verwendet werden.

Die Welle des Handhabungswerkzeugs kann eine Kupplung aufweisen. Mit einer Kupplung kann das Handhabungswerkzeug insbesondere in manuell oder motorisch angetriebene Instrumente eingesetzt werden.

Vorzugsweise ist die Kupplung als Schnellkupplung, also als werkzeuglos betätigbare Kupplung ausgebildet. Als Kupplungen kommen vorzugsweise im medizinischen, insbesondere dentalen, Bereich verwendete Standardkupplungen in Betracht.

Das Handhabungswerkzeug kann beispielsweise als in ein Instrument einsetzbares Bit ausgebildet sein.

### Kurzbeschreibung der Zeichnungen

Der Gegenstand der Erfindung soll im Folgenden bezugnehmend auf die Zeichnungen Fig. 1 bis Fig. 9 anhand eines Ausführungsbeispiels erläutert werden.
Fig. 1 ist eine Seitenansicht einer erfindungsgemäßen Kortikalisschraube.
Fig. 2 ist eine axiale Schnittansicht.
Fig. 3 ist eine Draufsicht auf die Kortikalisschraube von hinten.
Fig. 4 zeigt die Kortikalisschraube von vorne.
Bezugnehmend auf Fig. 5, welches die das Gewinde einhüllende Geometrie der Kortikalisschraube zeigt, soll die Ausgestaltung der Kortikalisschraube weiter erläutert werden.
Fig. 6 ist eine perspektivische Ansicht der Kortikalisschraube.
Fig. 7 zeigt in einer schematischen perspektivischen Ansicht ein beispielhaftes Handhabungswerkzeug für eine erfindungsgemäße Kortikalisschraube.
Fig. 8 ist eine schematische Ansicht der Düse einer Extrusionsmaschine, wie sie zur Herstellung einer erfindungsgemäßen Kortikalisschraube verwendet werden kann.
Fig. 9 zeigt eine alternative Ausführungsform eines Handhabungswerkzeugs, welches als Einsatz für ein Handstück ausgebildet ist.

### Detaillierte Beschreibung der Zeichnungen

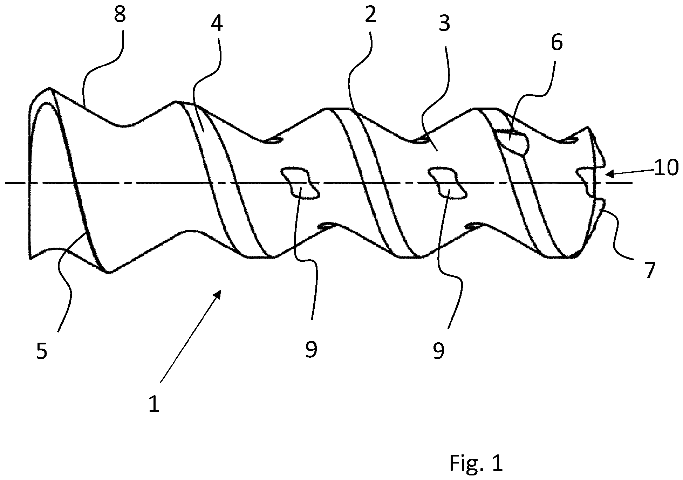

Fig. 1 zeigt in einer Seitenansicht ein Ausführungsbeispiel einer erfindungsgemäßen Kortikalisschraube 1.

Die Kortikalisschraube 1 umfasst ein Schraubengewinde 2, welches sich in diesem Ausführungsbeispiel von vorne bis zu einem Kopf 8 mit einem Kopfgewinde 5 erstreckt.

Die Zahnspitzen 4 des Schraubengewindes 2 sind abgeflacht, insbesondere eben ausgebildet, um die Klemmung der eingesetzten Kortikalisschraube 1 zu verbessern und die Beschädigung von Weichgewebe zu verringern. Der abgeflachte Abschnitt, der durch die Zahnspitzen 4 gebildet wird, kann insbesondere eine Länge von 0,3 bis 2 mm haben.

Das Schraubengewinde 2 geht übergangslos, also ohne Stufe, in einen ansonsten konisch, insbesondere kegelstupfförmig, ausgebildeten Kopf 8 über.

Sowohl das Schraubengewinde 2 als auch das so gebildete Kopfgewinde 5 sind in diesem Ausführungsbeispiel einzügig ausgebildet.

Das Schraubengewinde 2 hat eine größere Steigung als das Kopfgewinde 5.

Insbesondere ist die Steigung des Schraubengewindes 2 5 bis 20 %, besonders bevorzugt 8 bis 15 %, größer als die Steigung des Kopfgewindes 5.

So verklemmt sich die Kortikalisschraube 1 am Ende des Einschraubvorgangs.

Die Steigung des Schraubengewindes 2 beträgt vorzugsweise 2 bis 5 mm.

Die Kortikalisschraube 1 hat vorzugsweise eine Länge von 6 bis 20 mm, besonders bevorzugt von 8 bis 14 mm.

Die Länge des Kopfes 8 beträgt vorzugsweise 1/2 bis 1/10 der Gesamtlänge der Kortikalisschraube 1, besonders bevorzugt 1/4 bis 2/5.

Der Durchmesser der Kortikalisschraube 1 im Bereich des Schraubengewindes 2 beträgt vorzugsweise 2 bis 5 mm, besonders bevorzugt 2,5 bis 3,5 mm.

Die Kortikalisschraube 1 hat in diesem Ausführungsbeispiel eine Spitze 7 mit einem vorne offenen Kanal 10.

Der Kanal 10 überschneidet sich partiell mit dem Zahngrund 3 des Gewindes 2.

Hierdurch entstehen im Zahngrund 3 umlaufend verteilte Durchgänge 9.

Die Durchgänge 9 ermöglichen einen Druckausgleich sowohl in Bezug auf Gase als auch hinsichtlich osmotischer Drücke.

Die Spitze 7 der Kortikalisschraube 1 ist selbstschneidend ausgebildet.

Dies ist bei diesem Ausführungsbeispiel durch zumindest einen Anschliff 6 des ersten vorderen Gewindegangs realisiert.

Der Zahngrund 3 ist abgerundet ausgebildet, insbesondere mit einem Radius von 0,5 bis 1 mm.

Dies erleichtert ebenfalls das Verklemmen des Implantats und reduziert die Beschädigung von Weichgewebe.

Die Zahntiefe beträgt vorzugsweise 0,5 bis 0,8 mm.

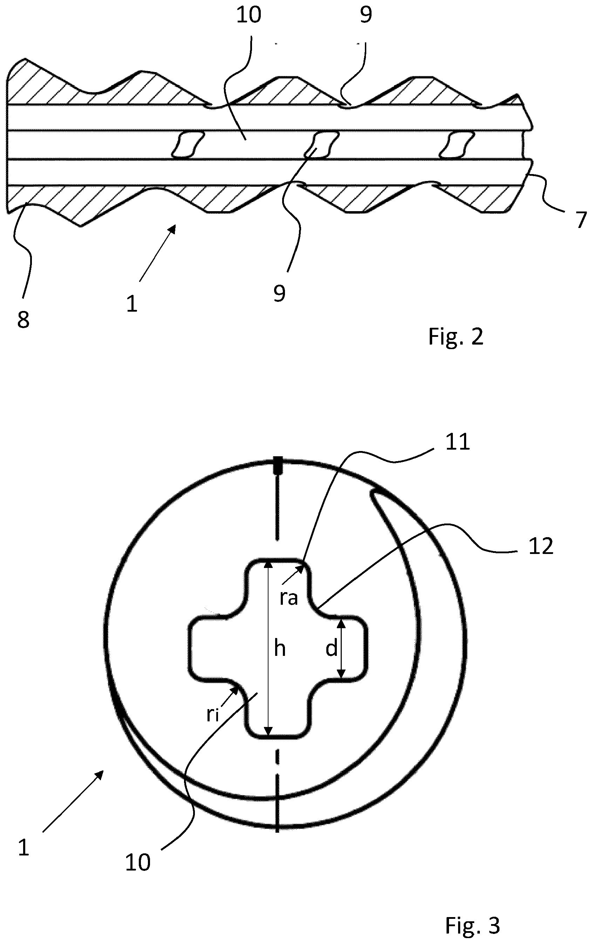

Fig. 2 zeigt in einer axialen Schnittansicht der in Fig. 1 dargestellten Kortikalisschraube 1.

Zu erkennen ist insbesondere der Kanal 10, welcher sich vom hinteren Ende der Kortikalisschraube bis zum vorderen Ende der Kortikalisschraube 1 erstreckt.

Der Kanal ist, wie in der Draufsicht auf die Rückseite der Kortikalisschraube gemäß Fig. 3 dargestellt, kreuzförmig ausgebildet, so dass sich, wie in Fig. 2 zu erkennen, in jedem Gewindegang vier um jeweils 90° verteilte Durchgänge 9 ergeben, da dass sich der Gewindegrund mit dem Kanal 10 überschneidet.

In der Draufsicht auf die Rückseite der Kortikalisschraube 1 ist insbesondere die kreuzförmige Ausgestaltung des Kanals 10 zu erkennen.

Der Kanal 10 hat vorzugsweise eine Höhe h (entspricht der Breite) von 1,3 bis 2,5 mm, besonders bevorzugt von 1,5 bis 2,1 mm.

Die Höhe h bzw. die Breite des Kanals 10 und/oder dessen größter Durchmesser beträgt vorzugsweise mindestens 20 % vorzugsweise mindestens 40 % des Außendurchmessers des Gewindes, durch welches sich der Kanal erstreckt.

Der Kanal 10 besteht, im Querschnitt gesehen, aus zwei um 90° zueinander verdrehten Rechtecken mit identischem Querschnitt und ist zentrisch in der Kortikalisschraube 1 angeordnet.

Vorzugsweise sind sowohl die Außenkanten 11 als auch die Innenkanten 12 abgerundet.

Der Radius der Außenkanten rₐ und/oder der Radius der Innenkanten rᵢ kann insbesondere 0,5 bis 1 mm betragen.

Die Höhe h des Kanals beträgt vorzugsweise das 2 bis 4fache, besonders bevorzugt das 2,5 bis 3,5fache, der Dicke d der Arme des Kreuzes.

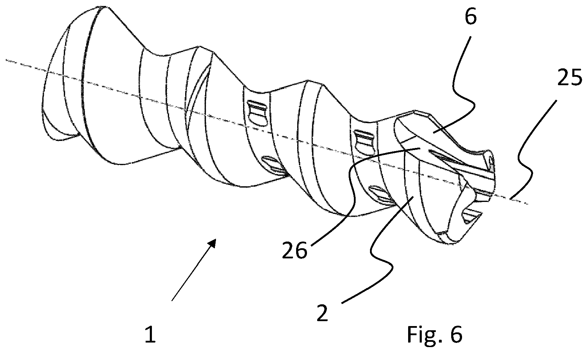

Durch diese Ausgestaltung wird ein leichtes Einführen des Handhabungswerkzeugs (siehe Fig. 6) ermöglicht. Gleichzeitig stabilisiert das Handhabungswerkzeug die gesamte Kortikalisschraube 1 beim Eindrehen und die Drehmomentübertragung wird über den gesamten Kanal 10 verteilt.

Durch den Kanal 10 wird des Weiteren das Gewicht des Implantats 1 deutlich reduziert. Hierdurch muss weniger bioresorbierbares Material, insbesondere weniger Magnesium abgebaut werden, was sowohl die Gasbildung als auch die aufgrund der Bildung von Metallsalzen entstehenden osmotischen Drücke reduziert.

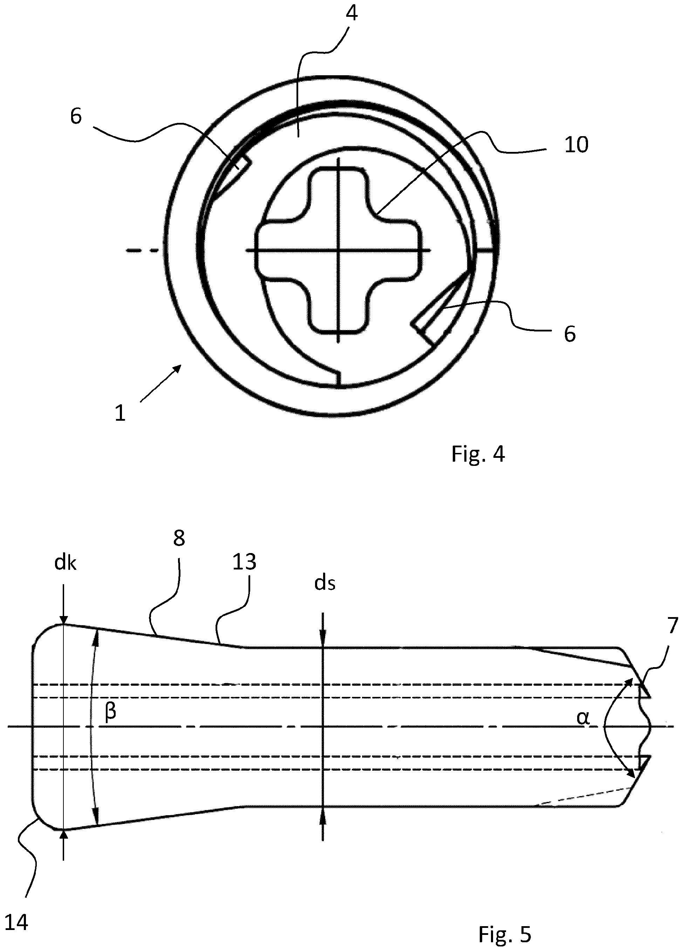

Fig. 4 ist eine Draufsicht auf die Spitze der Kortikalisschraube 1.

Zu erkennen ist, dass der Kanal 10 durchgängig von hinten nach vorne verläuft.

Weiter ist zu erkennen, dass die Zahnspitze 4 des ersten Gewindegangs in diesem Ausführungsbeispiel zwei sich gegenüberliegende Anschliffe 6 aufweist, die eine selbstschneidende Ausgestaltung bewirken.

Fig. 5 zeigt die das Gewinde einhüllende Kontur der Kortikalisschraube bzw. deren Form vor beispielsweise dem spanenden Formen des Gewindes.

Zu erkennen ist, dass die Grenze 13 vom Schraubengewinde 2 zum Kopf 8 übergangslos erfolgt.

Der mit einem Kopfgewinde versehene Kopf 8 ist konisch ausgebildet.

Der Durchmesser der Kortikalisschraube 1 im Bereich des Gewindes dₛ beträgt vorzugsweise 2 bis 5 mm.

Der maximale Durchmesser der Kortikalisschraube am Ende des konischen, insbesondere kegelstumpfförmigen Kopfes 8 dₖ ist vorzugsweise 20 bis 40 %, besonders bevorzugt 25 bis 35 %, größer als der Außendurchmesser des Schraubengewindes dₛ.

Die hintere Ecke 14 der Kortikalisschraube ist vorzugsweise abgerundet.

Der konisch ausgebildete Kopf hat vorzugsweise einen spitzen Winkel β von 90 bis 150°, besonders bevorzugt von 110 bis 130°.

Die Kortikalisschraube ist vorzugsweise an der Spitze 7 spitz zulaufend ausgebildet. Der Spitzenwinkel α beträgt vorzugsweise 90 bis 150°, besonders bevorzugt 110 bis 130°.

Fig. 6 ist eine perspektivische Ansicht der zuvor dargestellten Kortikalisschraube 1.

Gut zu erkennen ist, dass der Anschliff 6 als ausgeschnittenes Segment der Kortikalisschraube 1 ausgebildet ist. Der Anschliff 6 erstreckt sich über den ersten Gewindegang des Schraubengewindes 2 und läuft sodann aus.

Der Anschliff 6 ist insbesondere als kuchenstückartiger gewinkelter Einschnitt ausgebildet, der nach hinten ausläuft.

Die Fläche 6, die durch den Anschliff gebildet wird, ist vorzugsweise im Wesentlichen zur Mittelachse 25 zeigende radial verlaufende Fläche ausgebildet.

Die durch den Anschliff gebildete angrenzende axial verlaufende Fläche 26 schließt vorzugsweise mit einer Fläche des Anschliffs einen Winkel von 50° bis 120° ein, insbesondere einen Winkel von 90°.

So kann der Anschliff beispielsweise mit einem Walzenfräser geformt werden, in dem dieser von der Spitze ausgehend ein rechtwinkliges Segment abträgt, welches nach der Zahnspitze des ersten Gewindegangs ausläuft.

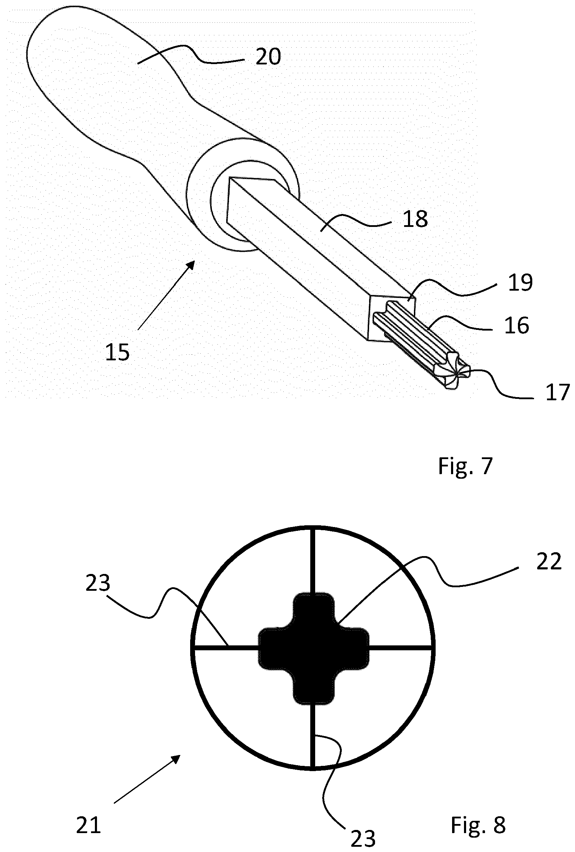

Fig. 7 zeigt in einer perspektivischen Darstellung ein schematisch dargestelltes Handhabungswerkzeug 15 für die zuvor dargestellte Kortikalisschraube.

Das Handhabungswerkzeug 15 ist als Schraubendreher ausgebildet und umfasst einen Griff 20 mit einer Welle 16.

Die Welle 16 ist kreuzförmig ausgebildet. Die Form der Welle 16 entspricht (bis auf Toleranzen) der Form des Kanals der Kortikalisschraube.

An der Spitze 17 ist die Welle 16 als Bohrer ausgebildet und dient in dem Bereich des Kanals der Kortikalisschraube dem Vorbohren.

An die Welle 16 grenzt in diesem Ausführungsbeispiel ein dickerer Schaft 18 an, welcher einen Anschlag 19 für die aufgesetzte Kortikalisschraube bildet.

So kann die Kortikalisschraube in einfacher Weise auf den Schaft 16 gesetzt und mittels des Handhabungswerkzeugs 15 eingedreht werden.

Nach vollständigem Eindrehen kann das Handhabungswerkzeug 15 einfach herausgezogen werden.

Es versteht sich, dass das Handhabungswerkzeug in einer anderen Ausführungsform (nicht dargestellt) beispielsweise auch ein austauschbares Bit umfassen kann, welches der Aufnahme der Kortikalisschraube dient.

Fig. 8 ist eine schematische Ansicht der Düse 21 einer Extrusionsmaschine, wie sie zur Herstellung eines Halbzeugs für eine erfindungsgemäße Kortikalisschraube verwendet werden kann.

Die Düse 21 umfasst einen Einsatz 22, der mittels zumindest eines Stegs 23 mit der Wand der Düse 21 verbunden ist.

Die Form des Einsatzes 21 entspricht dem Kanal der Kortikalisschraube. Ein Stab (nicht dargestellt) aus einer Magnesiumlegierung wird durch die Düse 21 extrudiert. Durch entsprechende, an die Schmelztemperatur der Legierung angepasste Druck- und/oder Temperaturführung verflüssigt sich das Material im Bereich des Einsatzes, so dass es um den zumindest einen Steg 23 fließt.

Das so hergestellte Halbzeug (nicht darstellt) kann sodann zur Herstellung der Kortikalisschraube verwendet werden, z.B. indem durch spanende Bearbeitung zunächst die in Fig. 5 dargestellte Kontur und sodann das Gewinde mit den Anschliffen hergestellt wird.

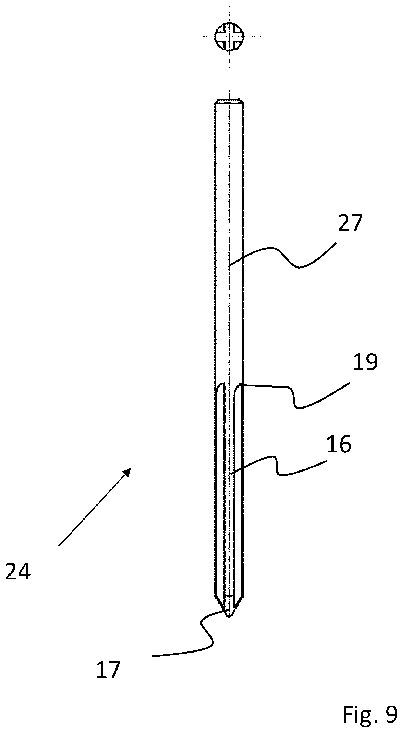

Fig. 9 zeigt in einer Seitenansicht sowie in einer Draufsicht auf die Spitze ein weiteres Ausführungsbeispiel eines Handhabungswerkzeugs 24, welches als Einsatz für ein medizinisches Instrument, insbesondere ein motorisch betriebenes Instrument (nicht dargestellt), ausgebildet ist.

Auch diese Ausführungsform umfasst eine kreuzförmige Welle 16 mit einer Spitze 17, die dem Antrieb des Implantats 1 dient.

Im Unterschied zu der in Fig. 7 dargestellten Ausführungsform ist der Anschlag 19 dadurch bereitgestellt, dass die kreuzförmige Welle 16 im Bereich des Anschlags 19 zu einem anderen Querschnitt, insbesondere zu einem kreisförmigen Querschnitt, übergeht.

Der Übergang verläuft in diesem Ausführungsbeispiel nicht senkrecht, sondern schräg zur Mittelachse. Durch die so schräg zur Mittelachse 27 gebildeten Flächen verklemmt sich die Kortikalisschraube im Bereich des Anschlags 19 und ist so nach dem Aufsetzen gegen unabsichtliches Lösen gesichert.

Mit dem Schaft 18 kann das Handhabungswerkzeug in ein medizinisches Instrument eingesetzt werden.

Durch die Erfindung konnte eine bioresorbierbare Kortikalisschraube bereitgestellt werden, welche sich leichter und sicherer einschrauben lässt, als auch, bei welcher das Gewicht der Schraube minimiert werden kann, was insbesondere bei Implantaten aus Magnesium und Magnesiumlegierungen entstehende Gasdrücke und osmotische Drücke reduziert.

### Bezugszeichenliste

- 1: Kortikalisschraube
- 2: Schraubengewinde
- 3: Zahngrund
- 4: Zahnspitze
- 5: Kopfgewinde
- 6: Anschliff
- 7: Spitze
- 8: Kopf
- 9: Durchgang
- 10: Kanal
- 11: Außenkante
- 12: Innenkante
- 13: Grenze von Gewinde zum Kopfgewinde
- 14: Ecke
- 15: Handhabungswerkzeug
- 16: Welle
- 17: Spitze
- 18: Schaft
- 19: Anschlag
- 20: Griff
- 21: Düse
- 22: Einsatz
- 23: Steg
- 24: Handhabungswerkzeug zum Einsetzen
- 25: Mittelachse
- 26: Fläche
- 27: Mittelachse des Handhabungswerkzeugs

## Patentansprüche

1. Kortikalisschraube (1) aus einem bioresorbierbaren Material, umfassend ein Schraubengewinde (2) sowie einen Kopf (8) mit einem Antrieb, wobei sich der Antrieb zumindest abschnittsweise durch das Schraubengewinde (2) erstreckt, und wobei das Schraubengewinde (2) übergangslos in einen konischen Kopf (8) mit einem Kopfgewinde (5) übergeht, wobei ein letzter Gewindezug des Schraubengewindes (2)in einen Gewindezug des Kopfgewindes (5)übergeht, und wobei die Zahnspitzen (4) des Schraubengewindes (2) abgeflacht ausgebildet sind, **dadurch gekennzeichnet, dass** die Kortikalisschraube aus Magnesium oder einer Magnesiumlegierung besteht, und dass das Schraubengewinde (2) eine größere Steigung als ein Kopfgewinde (5) aufweist.

2. Kortikalisschraube (1) nach dem vorstehenden Anspruch, **dadurch gekennzeichnet, dass** die Zahnspitzen (4) des Schraubengewindes (2) auf einer Länge von 0,3 bis 2 mm abgeflacht sind.

3. Kortikalisschraube (1) nach dem vorstehenden Anspruch, **dadurch gekennzeichnet, dass** der Antrieb als ein von einem hinteren Ende bis zu einem vorderen Ende durchgehender Kanal (10) ausgebildet ist, welcher ein Formschlusselement für ein Handhabungswerkzeug (15) ausbildet.

4. Kortikalisschraube (1) nach einem der vorstehenden Ansprüche, **dadurch gekennzeichnet, dass** die Kortikalisschraube (1) eine Beschichtung umfasst, insbesondere eine Magnesiumfluoridbeschichtung und/oder eine Polymerbeschichtung, vorzugsweise eine Beschichtung mit einer Dicke von weniger als 5 µm, vorzugsweise weniger als 1 µm, insbesondere eine 0,2 bis 0,8 µm dicke Polymerbeschichtung.

5. Kortikalisschraube (1) aus einem bioresorbierbaren Material, nach einem der vorstehenden Ansprüche, umfassend ein Schraubengewinde (2) sowie einen Kopf (8), wobei die Kortikalisschraube (1) einen sich axial erstreckenden Kanal (10) aufweist, **dadurch gekennzeichnet, dass** das Schraubengewinde (2) der Kortikalisschraube (1) Durchgänge zum Kanal (10) aufweist.

6. Kortikalisschraube (1) nach dem vorstehenden Anspruch, **dadurch gekennzeichnet, dass** die Durchgänge als Überschneidungen des Kanals (10) mit dem Zahngrund des Schraubengewindes (2) ausgebildet sind.

7. Kortikalisschraube (1) nach einem der beiden vorstehenden Ansprüche, **dadurch gekennzeichnet, dass** der Kanal (10) als Antrieb für die Kortikalisschraube (1) ausgebildet ist.

8. Kortikalisschraube (1) nach einem der vorstehenden Ansprüche, **dadurch gekennzeichnet, dass** die Kortikalisschraube (1) eine Länge von 6 bis 20 mm und/oder einen Durchmesser von 2 bis 5 mm und/oder eine gegenüber dem Außendurchmesser 1 bis 20 fache, vorzugsweise 5 bis 10 fache Länge aufweist, und/oder dass die Kortikalisschraube (1) aus einer Magnesiumlegierung mit einer Zugfestigkeit Rm (nach DIN EN ISO 6892-1, Version 2017-02) von über 250 MPa, insbesondere über 350 MPa, und/oder unter 500 MPa, insbesondere unter 450 MPa, besteht.

9. Set mit einer Kortikalisschraube (1) nach einem der vorstehenden Ansprüche und einem Handhabungswerkzeug (15), wobei das Handhabungswerkzeug (15) eine Welle umfasst, welche an den als Formschlusselement ausgebildeten Kanal (10) angepasst ist.

10. Set nach Anspruch 9, **dadurch gekennzeichnet, dass** die Spitze (7) der Welle (16) als Bohrer ausgebildet ist und/oder dass die Welle (16) eine Kupplung aufweist, insbesondere eine Schnellkupplung.

## Claims

1. A corticalis screw (1) consisting of a bioresorbable material,
comprising a screw thread (2)
as well as a head (8) with a drive, wherein the drive extends through the screw thread (2) at least in some sections, and wherein the screw thread (2) merges in a transition-free manner into a conical head (8) with a head thread (5), wherein a last thread flight of the screw thread (2) merges into a thread flight of the head thread (5), and wherein the tooth tips (4) of the screw thread (2) are formed to be flattened, **characterized in that** the corticalis screw consists of magnesium or a magnesium alloy, and that the screw thread (2) has a larger pitch than a head thread (5).

2. The corticalis screw (1) according to the preceding claim, **characterized in that** the tooth tips (4) of the screw thread (2) are flattened on a length of 0.3 to 2 mm.

3. The corticalis screw (1) according to the preceding claim, **characterized in that** the drive is formed as a channel (10), which is continuous from a rear end to a front end, and which forms a positive-locking element for a handling tool (15) .

4. The corticalis screw (1) according to any one of the preceding claims, **characterized in that** the corticalis screw (1) comprises a coating, in particular a magnesium fluoride coating and/or a polymer coating, preferably a coating with a thickness of less than 5 µm, preferably less than 1 um, in particular a polymer coating with a thickness of 0.2 to 0.8 um.

5. A corticalis screw (1) consisting of a bioresorbable material, according to any one of the preceding claims, comprising a screw thread (2) as well as a head (8), wherein the corticalis screw (1) has an axially extending channel (10), **characterized in that** the screw thread (2) of the corticalis screw (1) has passages to the channel (10).

6. The corticalis screw (1) according to the preceding claim, **characterized in that** the passages are formed as overlappings of the channel (10) with the tooth base of the screw thread (2).

7. The corticalis screw (1) according to any one of the two preceding claims, **characterized in that** the channel (10) is formed as drive for the corticalis screw (1).

8. The corticalis screw (1) according to any one of the preceding claims, **characterized in that** the corticalis screw (1) has a length of 6 to 20 mm and/or a diameter of 2 to 5 mm and/or a 1 to 20-times, preferably 5 to 10-times length compared to the outer diameter, and/or that the corticalis screw (1) consists of a magnesium alloy with a tensile strength Rm (according to DIN EN ISO 6892-1, version 2017-02) of above 250 MPa, in particular above 350 MPa, and/or below 500 MPa, in particular below 450 MPa.

9. A set with a corticalis screw (1) according to any one of the preceding claims and a handling tool (15), wherein the handling tool (15) comprises a shaft, which is adapted to the channel (10), which is formed as positive-locking element.

10. The set according to claim 9, **characterized in that** the tip (7) of the shaft (16) is formed as drill and/or that the shaft (16) has a coupling, in particular a quick coupling.

## Revendications

1. Vis corticale (1) en matériau biorésorbable,
comprenant un filet de vis (2) ainsi qu'une tête (8) avec un entraînement, l'entraînement s'étendant au moins par sections à travers le filet de vis (2), et le filet de vis (2) se transformant sans transition en une tête conique (8) avec un filet de tête (5), un dernier filetage du filet de vis (2) se transformant en un filetage du filet de tête (5), et les pointes dentées (4) du filet de vis (2) étant aplaties, **caractérisée en ce que** la vis corticale est composée de magnésium ou d'un alliage de magnésium, et **en ce que** le filet de vis (2) présente un pas plus grand qu'un filet de tête (5).

2. Vis corticale (1) selon la revendication précédente, **caractérisée en ce que** les pointes dentées (4) du filet de vis (2) sont aplaties sur une longueur de 0,3 à 2 mm.

3. Vis corticale (1) selon la revendication précédente, **caractérisée en ce que** l'entraînement est conçu sous forme de canal (10) s'étendant d'une extrémité arrière à une extrémité avant, lequel constitue un élément de complémentarité de forme pour un outil de manipulation (15) .

4. Vis corticale (1) selon l'une des revendications précédentes, **caractérisée en ce que** la vis corticale (1) comprend un revêtement, en particulier un revêtement de fluorure de magnésium et/ou un revêtement polymère, préférablement un revêtement d'une épaisseur inférieure à 5 um, préférablement inférieure à 1 um, en particulier un revêtement polymère d'une épaisseur de 0,2 à 0,8 µm.

5. Vis corticale (1) en matériau biorésorbable, selon l'une des revendications précédentes, comprenant un filet de vis (2) ainsi qu'une tête (8), la vis corticale (1) présentant un canal (10) qui s'étend axialement, **caractérisée en ce que** le filet de vis (2) de la vis corticale (1) comporte des passages vers le canal (10).

6. Vis corticale (1) selon la revendication précédente, **caractérisée en ce que** les passages sont conçus comme des intersections du canal (10) avec le fond denté du filet de vis (2).

7. Vis corticale (1) selon l'une des deux revendications précédentes, **caractérisée en ce que** le canal (10) est conçu comme un entraînement pour la vis corticale (1).

8. Vis corticale (1) selon l'une des revendications précédentes, **caractérisée en ce que** la vis corticale (1) présente une longueur de 6 à 20 mm et/ou un diamètre de 2 à 5 mm et/ou une longueur 1 à 20 fois, préférablement 5 à 10 fois plus grande que le diamètre extérieur, et/ou **en ce que** la vis corticale (1) est composée d'un alliage de magnésium présentant une résistance à la traction Rm (selon DIN EN ISO 6892-1, version 2017-02) supérieure à 250 MPa, en particulier supérieure à 350 MPa, et/ou inférieure à 500 MPa, en particulier inférieure à 450 MPa.

9. Ensemble comprenant une vis corticale (1) selon l'une des revendications précédentes, et un outil de manipulation (15), l'outil de manipulation (15) comprenant un arbre qui est adapté au canal (10) conçu sous forme d'élément de complémentarité de forme.

10. Ensemble selon la revendication 9, **caractérisé en ce que** la pointe (7) de l'arbre (16) est conçue comme un foret, et/ou **en ce que** l'arbre (16) présente un raccord, en particulier un raccord rapide.
